# EUROPÄISCHE PATENTANMELDUNG

(11) **EP 2 837 352 A1**
(43) Veröffentlichungstag der Anmeldung: **18.02.2015**
(21) Anmeldenummer: 13180744.8
(22) Anmeldetag: 16.08.2013
(51) Int. Cl.: A61B 19/00

(54) **Zusatzvorrichtung für Ultraschall-Reinigungsgeräte, Steuer- und/oder Bedienvorrichtung für ein Ultraschallgerät, sowie Verfahren zur aktiven Bewegung zumindest eines Bereichs eines medizinischen Instruments**

(71) Anmelder: BANDELIN patent GmbH & Co. KG, 12207 Berlin (DE)
(72) Erfinder: Jung, Rainer, 13125 Berlin (DE); Möhricke, Jonas, 10319 Berlin (DE)
(74) Vertreter: Pfenning, Meinig & Partner GbR

(57) **Zusammenfassung**

Die Anmeldung offenbart eine Zusatzvorrichtung (100) für eine Wanne (2) eines Ultraschallgeräts (1), welche ein Gestell (110) und mindestens einen Adapter (121 bis 124) für ein medizinisches Instrument (170) aufweist.

Dabei weist der Adapter ein Gehäuse (130) und mindestens ein mittels eines Antriebs (160) gegenüber dem Gehäuse bewegbares und mit dem medizinischen Instrument koppelbares Kopplungselement (140, 142; 144, 146) umfasst, so dass eine Bewegung des Kopplungselements eine aktive Bewegung zumindest eines Bereichs des medizinischen Instruments bewirkt.

## Beschreibung

Die Erfindung betrifft eine Zusatzvorrichtung für ein Ultraschallgerät bzw. ein Ultraschall-Reinigungsgerät, eine Steuer- und/oder Bedienvorrichtung für ein Ultraschallgerät mit einer derartigen Zusatzvorrichtung, sowie ein Verfahren zur aktiven Bewegung zumindest eines Bereichs eines medizinischen Instruments in einem Ultraschall-Reinigungsgerät,

Bei der Reinigung von medizinischen Instrumenten durch niederfrequenten Ultraschall werden Fremdpartikel auf oder in medizinischen Instrumenten aufgrund spezifischer Ultraschallwirkmechanismen (wie Kavitation, Microstreaming, Jetbuilding, etc.) gelöst.

Hierbei werden insbesondere chirurgische Instrumente in einen Korb gelegt und anschließend in einer mit einer Flüssigkeit befüllten Wanne eines Ultraschall-Reinigungsgeräts eingebracht. Anschließend wird die Wanne mit Ultraschall beaufschlagt, so dass sich Fremdpartikel und Anhaftungen in kurzer Zeit lösen können. Optional wird dieser Prozess durch den Zusatz geeigneter Reinigungs- und/oder Desinfektionspräparate die der Flüssigkeit zugegeben werden unterstützt.

Eine weitere Anwendung ist aus dem Bereich der Laparoskopie bekannt. Hierbei können Fremdpartikel nicht nur an der Außenseite des Instruments, sondern auch in einem Lumen des Schaftes auftreten. Um hier eine reinigende Wirkung zu entfalten, sind Vorrichtungen wie beispielsweise das Produkt Sonomic der BANDELIN electronic GmbH & Co. KG bekannt. Bei diesem Produkt wird an das distale Ende des Schaftes ein Adapter angebracht, welcher es ermöglicht, Flüssigkeit vom proximalen zum distalen Ende des Schaftes zu ziehen, so dass sich die Wirkung des Ultraschalls auch im Inneren des Schaftlumens entfalten kann.

Prinzipiell kann eine derartige Reinigungstechnik auch bei Instrumenten aus dem Bereich der robotergestützten minimal-invasiven Chirurgie, wie zum Beispiel die EndoWrist^{®}-Produkte des daVinci-Systems von Intuitive Surgical Inc., angewandt werden.

Jedoch besteht hier weiterer Bedarf, die Reinigungswirkung für medizinische Instrumente zu verbessern.

Die Aufgabe wird gelöst gemäß einer Zusatzvorrichtung für Ultraschall-Reinigungsgeräte nach den Merkmalen des Anspruchs 1, einer Steuer- und/oder Bedienvorrichtung für ein Ultraschallgerät nach Anspruch 11, sowie ein Verfahren zur aktiven Bewegung eines medizinischen Instruments gemäß Anspruch 13.

Die Zusatzvorrichtung umfasst vorzugsweise ein Gestell und zumindest einen Adapter für ein medizinisches Instrument. Die Zusatzvorrichtung ist derart ausgebildet, dass diese in einer Wanne eines Ultraschallgeräts, vorzugsweise eines Ultraschall-Reinigungsgeräts Platz findet. Dabei ist es vorteilhaft, wenn Teile des Gestells aus der Wanne herausragen, um so eine vereinfachte Handhabung der Zusatzvorrichtung beim Einbringen und Ausbringen aus der Wanne zu gewährleisten.

Der Adapter für das medizinische Instrument umfasst ein Gehäuse und mindestens ein gegenüber dem Gehäuse bewegbares und mit dem medizinischen Instrument koppelbares Kopplungselement. Dieses Kopplungselement ist mittels eines Antriebs bewegbar. Eine Bewegung des Kopplungselements bewirkt bei eingelegtem medizinischem Instrument eine aktive Bewegung zumindest eines Bereichs des medizinischen Instruments.

Instrumente aus dem Bereich der Telemedizin (und weitere Instrumente) besitzen Mechaniken, durch welche es möglich ist, bestimmte Bewegungen des medizinischen Instruments durchzuführen. So kann beispielsweise bei einem endoskopartigen Instrument der Schaft und die Spitze zumindest in einer die Rotationsachse des Schaftes umfassenden Ebene rotiert werden. Weiterhin ist es möglich, dass das Instrument selbst Greifarme oder ähnliches besitzt, welche in einer weiteren Ebene, senkrecht zur vorgenannten Ebene rotierbar ist. Das in dem Adapter angeordnete Kopplungselement ist derart ausgebildet, dass es an eine Mechanik des medizinischen Instruments andocken kann und somit eine Bewegung des Kopplungselements in eine aktive Bewegung zumindest eines Bereichs des medizinischen Instruments umgesetzt wird. Das heißt, das Kopplungselement ist derart ausgebildet, dass eine Bewegung des Kopplungselements, wie beispielsweise eine Rotation des Schaftes des medizinischen Instruments, oder eine Bewegung der distalen Spitze des medizinischen Instrumentes bewirkt wird.

Damit das Kopplungselement zuverlässig an das medizinische Instrument gekoppelt werden kann, so dass eine Bewegung das Kopplungselement in eine aktive Bewegung eines Bereichs des medizinischen Instrumentes umgesetzt wird, kann der Adapter Ausnehmungen, Stege oder ähnliches umfassen, welche zu Stegen bzw. Ausnehmungen eines medizinischen Instruments korrespondieren. Auf diese Weise kann sichergestellt werden, dass das Kopplungselement an vordefinierten Punkten des medizinischen Instruments angreift, vorzugweise an Mechaniken des medizinischen Instruments ansetzt, welche für eine aktive Bewegung zumindest eines Bereichs des medizinischen Instruments notwendig sind.

Aufgrund der aktiven Bewegung des medizinischen Instruments durch die Zusatzvorrichtung, kann das medizinische Instrument während der Beaufschlagung der Wanne mit Ultraschall aktiv bewegt werden, so dass eine verbesserte Reinigungswirkung im Bereich der sich bewegenden Teile des medizinischen Instruments gewährleistet wird. Auf diese Weise wird gewährleistet, dass der Ultraschall auch bei eng aneinander anliegenden mechanischen bzw. beweglichen Teilen des Instruments seine volle Wirkung entfalten kann und Fremdpartikel zuverlässig von dem medizinischen Instrument, auch von verdeckten Teilflächen in z.B. Gelenken, entfernt werden.

In einer Ausführungsform der Zusatzvorrichtung umfasst der Adapter eine Fixierungsvorrichtung zum Fixieren des medizinischen Instruments. Zusätzlich oder optional zu dem bereits erwähnten Ausnehmungen oder Stegen des Adapters, kann eine Fixierungs- oder Verriegelungsvorrichtung vorgesehen sein, welche zumindest einen weiteren Bereich des medizinischen Instruments auf dem Adapter fixiert. Dabei ist die Fixierung dergestalt, dass das Kopplungselement der Zusatzvorrichtung an einem Punkt des medizinischen Instruments angreift, welcher eine aktive Bewegung eines Bereichs des medizinischen Instruments, welcher nicht durch die Fixierungsvorrichtung fixiert ist, bewirkt. Im Falle eines chirurgischen Robotikinstruments kann dies beispielsweise bedeuten, dass das proximale Ende des Instruments, welches zu Zwecken eines medizinischen Eingriffs mit einem Roboterantrieb verbunden wird, in den Adapter eingesetzt wird und durch die Fixiervorrichtung verriegelt wird. Zwar ist nunmehr das proximale Ende des medizinischen Instruments fixiert, jedoch ist das Kopplungselement des Adapters derart an das proximale Ende des medizinischen Instruments gekoppelt, dass sowohl der Schaft als auch die Instrumente am distalen Ende des medizinischen Instruments durch eine Bewegung des Kopplungselements aktiv bewegt werden.

In einer weiteren Ausführungsform umfasst die Zusatzvorrichtung an einer dem medizinischen Instrument zugewandten Oberfläche des Kopplungselements eine Nut oder einen Steg, welche(r) in einen mechanischen Formschluss mit einer Mechanik des zu reinigenden medizinischen Instruments gebracht werden kann. Ist die Mechanik des medizinischen Instruments beispielsweise durch einen Stift bzw. eine Nut gegeben, so ist die Nut bzw. der Steg des Kopplungselements dazu korrespondierend ausgebildet. Auf diese Weise kann eine Bewegung des Kopplungselements direkt auf die Mechanik des medizinischen Instruments übertragen werden.

In einer weiteren Ausführungsform ist das Kopplungselement federnd gelagert. Da das Kopplungselement bevorzugt derart ausgebildet ist, in eine Mechanik des medizinischen Instruments einzugreifen, die Mechanik des medizinischen Instruments und das Kopplungselement jedoch nicht in jeder Orientierung zueinander ineinandergreifen können, kann das Kopplungselement federnd gelagert sein. Dies bewirkt, dass beim Einlegen des medizinischen Instruments das Kopplungselement zunächst nach unten gedrückt wird, sofern es nicht mit der Mechanik des medizinischen Instruments in Eingriff steht, das heißt, dass eine Bewegung des Kopplungselements in eine aktive Bewegung eines Bereichs des medizinischen Instruments umgewandelt werden kann. Durch die federnde Lagerung kann zunächst das Kopplungselement bewegt werden, so dass es eine Position einnimmt, in welcher das Kopplungselement mit der Mechanik des medizinischen Instruments in Eingriff tritt. Dies kann beispielsweise dadurch passieren, dass ein Stift der Mechanik in eine Nut des Adapters eingreift. Erst durch den Eingriff überträgt sich eine Bewegung des Kopplungselements auf die Mechanik und wird so in eine aktive Bewegung des medizinischen Instruments umgewandelt. Durch das Einrutschen des Stiftes in die Nut drückt eine Federwirkung (oder eine vergleichbare Wirkung) das Kopplungselement in Richtung des medizinischen Instruments und bewirkt so einen Formschluss zwischen einer Mechanik des medizinischen Instruments und dem Kopplungselement.

In einer weiteren Ausführungsform ist das Kopplungselement derart durch einen Antrieb bewegbar, dass dieses rotiert wird. Dabei kann der Antrieb derart ausgebildet sein, dass das Kopplungselement abwechselnd in eine und anschießend in eine entgegengesetzte Richtung rotiert wird. Es sind jedoch auch Varianten mit einer linearen Bewegung des Kopplungselements bei entsprechenden medizinischen Instrumenten möglich.

In einer weiteren Ausführungsform der Erfindung ist die Zusatzvorrichtung bzw. das Kopplungselement mechanisch, hydraulisch oder pneumatisch mit einem Antrieb gekoppelt. Hierdurch kann erreicht werden, dass keinerlei elektrische Komponenten des Antriebs sich innerhalb der Wanne des Ultraschallgeräts befinden müssen. Dies vereinfacht die betriebliche Sicherheit und die Zuverlässigkeit der Zusatzvorrichtung. Das Kopplungselement kann demnach mechanische, hydraulische oder pneumatische Getriebe aufweisen, welche eine Verbindung an beispielsweise einen Elektromotor ermöglichen.

In einer weiteren Ausführungsform ist das Gestell derart ausgebildet, dass eine am Gestell angeordnete krafterzeugende Komponente des Antriebs, wie beispielsweise ein Elektromotor außerhalb der Wanne und der mindestens eine Adapter von der krafterzeugenden Komponente beabstandet innerhalb der Wanne angeordnet ist. Hierdurch sind keinerlei elektrische Komponenten der Wirkung des Ultraschalls ausgesetzt, obgleich die medizinischen Instrumente vollständig in der Wanne versenkbar sind. Dies erhöht die Langlebigkeit der Zusatzvorrichtung. Weiterhin kann, beispielsweise im Falle eines Elektromotors, auf weiterführende Sicherheitsmaßnahmen verzichtet werden, da keinerlei elektrische Komponenten in die Wanne bzw. in die mit dem Ultraschall beaufschlagte Flüssigkeit eingebracht wird.

In einer weiteren Ausführungsform ist das Gehäuse des Adapters der Zusatzvorrichtung gegenüber dem Gestell fixiert. Der Adapter ist gegenüber dem Gestell unbeweglich, so dass sich lediglich das Kopplungselement gegenüber dem Gestell bewegen kann. Auf diese Weise ist es möglich, einen Teil des medizinischen Instruments zu fixieren und über das Kopplungselement einen weiteren Bereich des medizinischen Instruments aktiv zu bewegen.

In einer weiteren Ausführungsform umfasst die Zusatzvorrichtung Öffnungen in dem Gehäuse des Adapters, so dass die Flüssigkeit, welche sich in der Wanne befindet, über den Adapter bis in das medizinische Instrument eindringen kann. Hierdurch wird die reinigende Wirkung auf sämtliche Bereiche des medizinischen Instruments verbessert.

In einer weiteren Ausführungsform ist die Zusatzvorrichtung mit einer Saug- und/oder Druckspülungsvorrichtung zur Spülung des medizinischen Instruments verbunden, wobei die Spülungsvorrichtung einen Spüladapter zur Kopplung an das medizinische Instrument umfasst. Zahlreiche Instrumente weisen in einem Schaft ein oder mehrere Lumen auf. Je nachdem, welcher Bereich des medizinischen Instruments in dem Adapter fixiert wird, kann es opportun sein, eine Druckspülung oder eine Saugspülung durch den Schaft vorzunehmen. Hierzu wird eine Saug- und/oder Druckspülungsvorrichtung mittels eines Spüladapters an das medizinische Instrument gekoppelt. In weiteren Ausführungsformen kann alternierend sowohl Flüssigkeit durch den Spüladapter gesogen oder durch diesen in das Instrument gedrückt werden. In weiteren Ausführungsformen ist es möglich, mehr als einen Spüladapter mit dem medizinischen Instrument zu koppeln, um so gleichzeitig in einigen Bereichen eine Saugspülung und in anderen Bereichen des Instruments eine Druckspülung durchzuführen.

Mithilfe der Saug- und/oder Druckspülungsvorrichtung ist es möglich, während der aktiven Bewegung des medizinischen Instruments auch die Lumen des medizinischen Instruments zu spülen, um so die Wirkung des Ultraschalls auch im Inneren der medizinischen Instrumente zu verbessern. Hierbei ist eine Steuerung vorgesehen, welche die aktive Bewegung des medizinischen Instruments, das heißt die Bewegung des Kopplungselements und die Saug- und/oder Druckspülungsvorrichtung steuert.

Ein weiterer Aspekt der Erfindung betrifft eine Steuer- und/oder Bedienvorrichtung für ein Ultraschallgerät, welches zur Steuerung der Zusatzvorrichtung konfiguriert ist. Die Steuerung der Steuer- und/oder Bedienvorrichtung kann dabei eine Steuerungseinheit und eine Speichereinheit umfassen. Die Steuerungseinheit kann beispielsweise programmierbare Field-Arrays, Mikroprozessoren, Mikrocontroller oder ähnliche Vorrichtungen umfassen, welche dazu ausgebildet sind Instruktionen, beispielsweise eines Programmcodes, umzusetzen und hierdurch die Steuerung von Motoren, Pumpen oder ähnlichem zu bewirken. Die Steuer- und/oder Bedienvorrichtung ist dabei derart ausgebildet, dass eine regelmäßige periodische Bewegung des mindestens einen Kopplungselements durch den Antrieb ermöglicht wird. Dies kann beispielsweise bedeuten, dass das Kopplungselement für einen vorbestimmten Zeitraum nach links gedreht oder nach vorne bewegt wird, und für einen weiteren vorbestimmten Zeitraum in die entgegengesetzte Richtung bewegt wird. Die hierzu notwendigen Instruktionen können beispielsweise als Software in der Speichereinheit der Steuer- und/oder Bedienvorrichtung abgelegt werden. Bei der Speichereinheit kann es sich um volatilen oder nicht-volatilen Speicher handeln. Bevorzugt kommt ein Flash-Speicher zur Anwendung.

Weitere Programme zur Bewegung des Kopplungselements können ebenfalls als Software abgelegt werden. Hierdurch kann die jeweils gewünschte Bewegung des Koppelelements bewirkt werden. Die Steuer- und/oder Bedieneinheit kann den Antrieb umfassen oder kann mittels einer Daten- und/oder Stromverbindung mit einem externen, an der Zusatzvorrichtung angeordneten Antrieb verbunden sein. Hierzu ist einer Ausführungsform vorgesehen, dass ein Antrieb der Zusatzvorrichtung mittels eines Kabels und einer Steckverbindung zum einen durch die Steuer- und/oder Bedienvorrichtung mit Strom versorgt wird und zum anderen der Antrieb durch die Steuervorrichtung angesteuert wird.

In einer weiteren Ausführungsform umfasst die Steuer- und/oder Bedienvorrichtung die Spülungsvorrichtung, welche über einen Spüladapter mit dem medizinischen Instrument verbunden werden kann. Dabei ist die Steuer-und/oder Bedienvorrichtung derart konfiguriert, dass diese nicht nur den Bewegungsablauf des Kopplungselements regelt, sondern optional hierzu auch die Spülvorgänge eines Lumens des medizinischen Instrumentes steuert. So kann beispielsweise bei einer Bewegung des Kopplungselements in einer ersten Richtung eine Saugspülung des Instruments und bei dem Bewegen des Kopplungselements in einer zweiten, der ersten entgegengesetzten Richtung eine Druckspülung veranlasst werden. Alternativ kann über eine gleichzeitig aufgebrachte Druck- und -Saugspülung, welche jeweils über verschiedene Adapter umgesetzt wird, ein Flüssigkeitskreislauf innerhalb der Lumen des medizinischen Instruments erwirkt werden. Der Steuer- und/oder Bedienvorrichtung kommt hierbei die Aufgabe zu, die Spülvorgänge und die aktive Bewegung des medizinischen Instruments aufeinander abzustimmen und somit den größtmöglichen Reinigungseffekt zu erzielen.

In einer weiteren Ausführungsform umfasst die Steuer- und/oder Bedienvorrichtung ein berührungsempfindliches Display. Auf dem berührungsempfindlichen Display können verschiedene Piktogramme für das Abrufen der unterschiedlichen Funktionen der Steuer- und/oder Bedienvorrichtung abgebildet werden. So ist es beispielweise möglich, nur die Spülvorrichtung, nur die aktive Bewegung oder beide Programme gleichzeitig zu starten. Auch die Kontrolle der Ultraschallerzeuger zum Beaufschlagen der Wanne mit Ultraschall wird durch die Steuer- und/oder Bedienvorrichtung durchgeführt. Somit kann die Steuer- und/oder Bedienvorrichtung für verschiedene Ultraschall-Reinigungsgeräte und deren Zubehör eingesetzt werden. Zudem können weitere Funktionalitäten und/oder Ergänzungen der Funktionalitäten durch eine Schnittstelle in die Steuervorrichtung eingespielt werden, so dass Aktualisierungen der Software und einer Erweiterung der Steuerung möglich ist.

Weitere Ausführungsbeispiele werden anhand der nachfolgenden Figuren erläutert.

Es zeigen:
- FIG. 1: eine Aufsicht eines Ultraschall-Reinigungsgerätes mit einer Zusatzvorrichtung;
- FIG. 2: Detailansicht der Zusatzvorrichtung der FIG. 1;
- FIG. 3a: weitere Detailansicht der Zusatzvorrichtung der FIG. 1;
- FIG. 3b: Beispiel einer Fixiervorrichtung eines Adapters; und
- FIG. 4: Steuerungseinheit und Spülvorrichtung einer geöffneten Steuer- und/oder Bedieneinheit.

Die FIG, 1 zeigt ein Ultraschall-Reinigungsgerät 1, welches eine Wanne 2 umfasst, die mit einer Flüssigkeit befüllt werden kann. Auf der Unterseite 3 der Wanne befinden sich Schallübertragungselemente (nicht dargestellt), welche Ultraschall auf die Innenwände der Wanne 2 übertragen und so Ultraschallwellen in die in der Wanne 2 befindliche Flüssigkeit übertragen. Bei dem dargestellten Ultraschall-Reinigungsgerät handelt es sich um ein Einbaugerät, welches in die Oberfläche einer Ablage 4 eingelassen ist.

In der Wanne 2 befindet sich eine Zusatzvorrichtung 100, welche in den nachfolgenden Figuren näher erläutert wird. Die Zusatzvorrichtung 100 umfasst ein Gestell 110, welches über die Oberkante der Wanne 2 hinausragt. Am unteren Ende des Gestells 110 befindet sich eine Adapterbank 120, welche derart angeordnet ist, dass diese in die Wanne 2 eingelassen werden kann. Ist die Wanne 2 nun mit Flüssigkeit befüllt, bedeckt die Flüssigkeit die Adapterbank 120 und etwaige darauf angeordnete medizinische Instrumente vollständig.

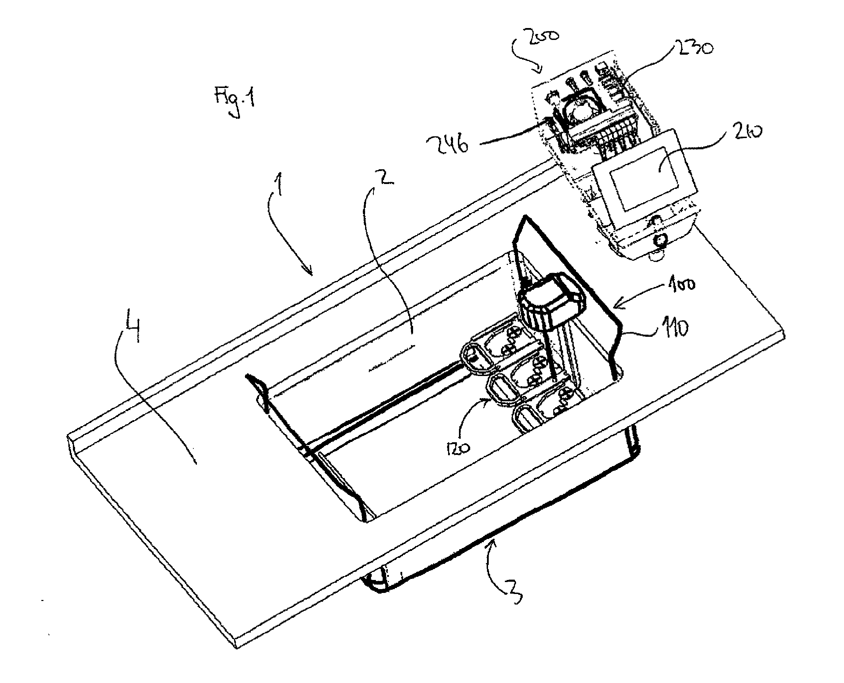

Die Steuer- und/oder Bedieneinheit 200 kann über eine Kabelverbindung mit einem Antrieb der Zusatzvorrichtung verbunden werden. Über das Kabel wird sowohl ein Motor des Antriebs mit Strom versorgt, als auch angesteuert, so dass dieser Bewegungen ausführt. Die Steuerung der Steuer- und Bedieneinheit 200 durch einen Benutzer wird über das berührungsempfindliche Display 210 vorgenommen. Es kann sich dabei um einen kapazitiven oder resistiven berührungsempfindlichen Bildschirm handeln. Weiterhin ist in der Darstellung der FIG. 1 eine Pumpe 246 zu erkennen. Weiterhin ist eine Steuerungsplatine 230 mit darauf angeordneten Prozessoren und Speichereinheiten dargestellt.

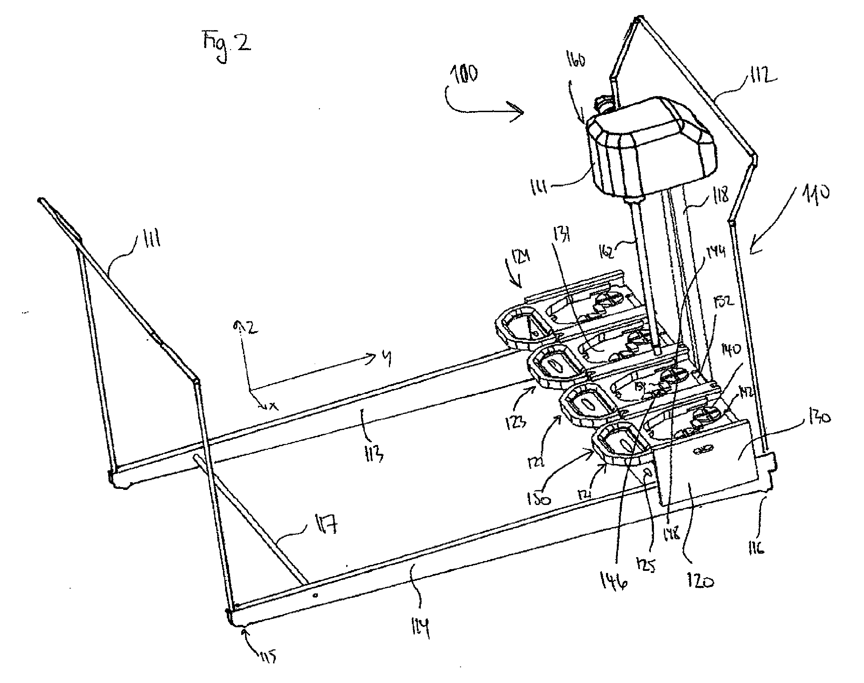

In der FIG. 2 ist die Zusatzvorrichtung 100 ohne die sie in der FIG. 1 umgebenden Wanne 2 dargestellt. Das Gestell 110 umfasst zwei in der x-z-Ebene verlaufende Griffteile 111 und 112, mittels derer die Zusatzvorrichtung 100 aus und in die Wanne gestellt werden kann. Am unteren Ende der Griffe 111 und 112 befinden sich Schienen 113 und 114, welche die beiden Griffe miteinander verbinden. An einem Ende der Schienen 113 und 114 ist zudem eine Adapterbank 120 angeordnet, welche im vorliegenden Ausführungsbeispiel vier Adapter 121 bis 124 umfasst.

Das Gestell 110 kann aus Metall oder einem Kunststoff gefertigt sein. Auch ist es möglich, dass beispielsweise die Griffe 111 und 112 aus Metall und die Schienen 113 und 114 aus einem Kunststoff gefertigt werden. An den Schienen 113, 114 befinden sich zudem Erhebungen 115 und 116, so dass das Gestell nur punktweise mit der Wanne in Berührung ist. Die konstante Breite des Gestells wird über Querstreben 117 gewährleistet.

Die Adapterbank 120 umfasst ein Gehäuse 130, welches zugleich das Gehäuse für die vier Adapter 121 bis 124 bildet. Das Gehäuse ist aus Kunststoff gefertigt und umfasst Öffnungen 125 welche es erlauben, dass in der Wanne befindliche Flüssigkeit ins innere des Gehäuses 130 eindringen kann.

Der Adapter 121 umfasst mindestens ein gegenüber dem Gehäuse bewegliches Kopplungselement 140 bzw. 142. Die Kopplungselemente können aus Kunststoff, Edelstahl, Titan, anderen medizinischen Werkstoffen oder anderen chemisch beständigen bzw. wasserbeständigen Werkstoffe gefertigt sein und stehen über die Oberfläche 131 des Gehäuses 130 in z-Richtung hinaus. Die beweglichen Kopplungselemente 140 und 142 sind in der z-Richtung federnd gelagert. Die Kopplungselemente sitzen auf einer in der Darstellung der FIG. 2 nicht erkennbaren Welle, welche über eine Mechanik, eine Hydraulik oder eine Pneumatik angetrieben wird. Die Welle ist an einem Boden des Gehäuses verankert und weist einen Federmechanismus auf, welche eine Federung des Kopplungselementes in z-Richtung erlaubt. Das heißt, das Kopplungselement kann zumindest um die Höhe, in der es über die Oberfläche 131 hinaussteht, in negativer z-Richtung eingedrückt werden. Hierdurch ist es möglich, dass das Kopplungselement nachgibt und die Drehung des Kopplungselements soweit voranschreitet, dass ein an einem medizinischen Instrument angeordneter Stift in die Nut des Kopplungselements eingreift und somit ein Formschluss erzeugt wird, so dass die Bewegung des Kopplungselements in eine aktive Bewegung des medizinischen Instruments umgesetzt wird. Die medizinischen Instrumente werden zudem mit einer Fixiervorrichtung 150 an ihrem proximalen Ende in der Adapterbank 120 fixiert. Hierdurch ist eine genaue Ausrichtung zwischen dem Kopplungselementen und der Mechanik der medizinischen Instrumente gegeben. An der Zusatzvorrichtung 100 ist zudem ein Antrieb 160 angeordnet, welcher am oberen Ende im Bereich des Griffs 112 angeordnet ist. Dieser umfasst ein Gehäuse 161, in welchem sich in diesem Falle ein Elektromotor befindet. An dem Elektromotor befindet sich eine Koppelstange 162, welche durch eine Öffnung in die Adapterbank 120 geführt wird und dort über das mechanische, pneumatische oder hydraulische Getriebe mit den einzelnen Kopplungselementen gekoppelt ist. Auf diese Weise kann eine Drehbewegung des Antriebs 160 unter anderem in eine Drehbewegung der Kopplungselemente umgesetzt werden. Das Gehäuse 161 ist im vorliegenden Beispiel über eine Strebe 118 mit der Adapterbank verbunden.

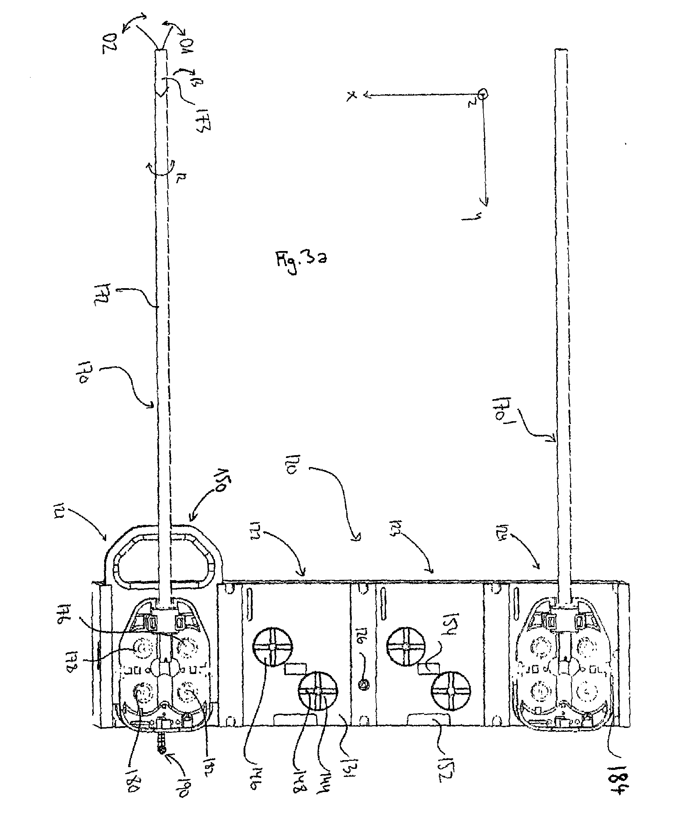

In der FIG. 3a ist eine Aufsicht auf die Adapterbank der FIG. 1 und 2 in der x-y-Ebene dargestellt. Neben der Adapterbank 120 sind eine Fixiervorrichtung 150, als auch zwei medizinische Instrumente 170 und 170' zu sehen, so dass die Interaktion zwischen dem Instrument 170 und den Kopplungselementen genauer erläutert werden kann. Das Instrument 170 umfasst einen Schaft 172, welcher entlang der Achse des Schaftes in der Richtung R rotierbar ist. Der Schaft 172 umfasst an seinem distalen Ende zudem eine bewegliche Spitze 173, welche in der y-z-Ebene in Richtung B rotierbar ist. An der Spitze 173 befinden sich zwei Arme einer Klemme 174, welche jeweils individuell geöffnet und geschlossen werden können, wobei ein Arm jeweils in der x-y-Ebene in einer Richtung 01 bzw. 02 bewegt werden. An seinem proximalen Ende umfasst das Instrument ein (in dieser Aufsicht aufgeschnittenes) Gehäuse, welches unter anderem vier Mechaniken 176, 178, 180 und 182 umfasst. Dabei bewirkt eine Rotation des Elements 176 ein Öffnen in der Richtung 02, die Mechanik 178 eine Rotation in Richtung 01, das Element 180 eine Rotation in der Richtung R und das Element 182 eine Rotation in der Richtung B. In dem hier dargestellten Ausführungsbeispiel umfasst jeder der Adapter der Adapterbank 120 zwei bewegliche Kopplungselemente 144 und 146, welche jeweils zwei kreuzförmig zueinander verlaufenden Nuten 148 umfasst. Die Nuten sind dabei derart gewählt, dass ein an der nicht dargestellten Unterseite der Mechaniken 176 bis 182 befindlicher Stift in den Nuten formschlüssig gehalten werden kann. Sobald der Stift in der Nut 148 formschlüssig gehalten ist, wird eine Bewegung des Kopplungselements 144 beispielsweise in eine Bewegung der Mechanik 182 und somit eine aktive Bewegung der Spitze 173 in der Richtung B umgesetzt.

Wie bereits zur FIG. 2 erwähnt, stehen die Kopplungselemente 140, 142, 144 und 146 in der z-Richtung über die Oberfläche 131 leicht hinaus. Wird nun das medizinische Instrument 170 in dem Adapter eingelegt, drücken die Stifte an der Unterseite der Mechanik des medizinischen Instruments zunächst auf den Bereich des Kopplungselements 144 bzw. 146, welcher die Nuten begrenzt. Hierdurch wird das Kopplungselement in z-Richtung nach unten gedrückt. Bei einer Drehung des Kopplungselements rutscht der Stift der Mechanik nun auf der die Nuten 148 begrenzenden Oberfläche entlang bis dieser in eine der Nuten eingreift. Hierdurch ist der Druck auf das federnd gelagerte Kopplungselement 144 reduziert und das Kopplungselement wird in positiver z-Richtung nach oben gedrückt. Hierdurch wird der Formschluss zwischen der Nut und dem Stift des medizinischen Instruments herbeigeführt.

Obgleich in dem hier gezeigten Ausführungsbeispiel lediglich zwei Kopplungselemente je medizinischem Instrument vorhanden sind, können in weiteren Ausführungsformen auch weniger, das heißt eins, oder mehr Kopplungselemente, das heißt drei bis vier, vorhanden sein, um sämtliche Bewegungen des distalen Bereichs des medizinischen Instruments aktiv herbeizuführen. Dabei kann die Bewegung des distalen Teils des medizinischen Instruments dergestalt sein, dass das Kopplungselement 144 zunächst um einen bestimmten Winkel im Uhrzeigersinn und anschließend um den gleichen Winkel gegen den Uhrzeigersinn bewegt wird. Hierdurch wird eine aktive Bewegung auf das medizinische Instrument übertragen, so dass die distale Spitze 173 über einen Winkelbereich von beispielsweise 180° abgeknickt werden kann und die Arme der Klemme über einen Bereich von ebenfalls nahezu 180° bewegt werden können,

Die Nuten 148 dienen unter anderem auch der Fixierung des medizinischen Instruments. In anderen Ausführungsbeispielen können auch Löcher oder Stege die Funktion der Nuten übernehmen. Die Form des Fixierungselements, welches am Kopplungselement angeordnet ist, hängt somit im Wesentlichen von den Mechaniken der medizinischen Instrumente ab. Besitzen die medizinischen Instrumente Stifte, so sind Nuten oder Löcher zu bevorzugen. Besitzen diese Löcher oder Nuten, werden Stege oder Zapfen auf dem beweglichen Kopplungselement verwendet.

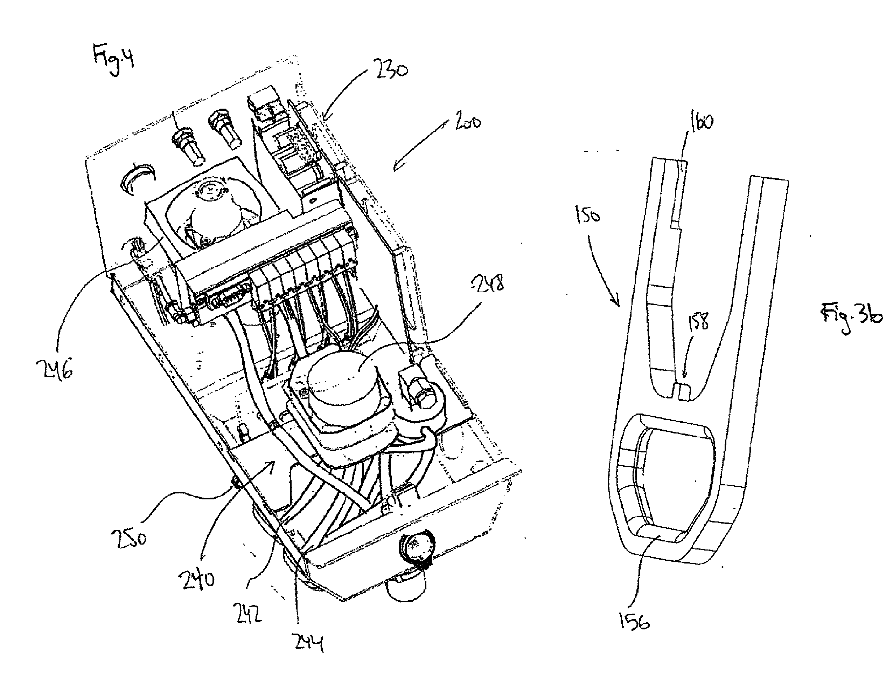

Neben den Nuten weist jeder Adapter noch weitere Fixierhilfen auf. So ist beispielsweise eine Aussparung 152 vorhanden, welche in die Oberfläche 131 eingelassen ist. In diesem Bereich befinden sich bei dem dargestellten medizinischen Instrument elektrische Kontakte, welche über die Unterseite des medizinischen Instruments leicht herausragen. Diese können in der Aussparung 152 ausgerichtet werden, so dass die Positionierung des medizinischen Instruments im Adapter vereinfacht wird. Desweiteren ist ein Steg 154 vorhanden, welcher ebenfalls in eine korrespondierende Aussparung der Unterseite des medizinischen Instruments eingreift. Die Fixiervorrichtung 150 weist zudem wie in der FIG. 3b erkennbar, eine Rastnase 158 auf, welche in eine korrespondierende Aussparung des medizinischen Instruments eingreift. Zudem umfasst die Fixiervorrichtung 150 einen Hinterschnitt 160, welcher mit einem Vorsprung 184 des medizinischen Instruments formschlüssig abschließt. Auf diese Weise ist das im Adapter 121 fixierte medizinische Instrument 170 in seiner Ausrichtung gegenüber dem Adapter 121 fixiert. Lediglich die Kopplung über das Kopplungselement 144 bzw. 146 und die entsprechende Mechanik 178 bzw. 182 bewirkt eine aktive Bewegung des distalen Endes des medizinischen Instruments. Weitere Teile oder Bereiche des medizinischen Instruments werden nicht durch die Kopplungselemente bewegt.

Die Fixiervorrichtung 150 umfasst zudem einen Griff 156, welcher in negativer y-Richtung gezogen, das medizinische Instrument 170 freigibt. Dieser Zustand ist beispielsweise im Adapter 124 zu erkennen. In weiteren Ausführungsbeispielen umfasst das Fixierungselement 150 einen Anschlag, so dass dieses nur, beispielsweise, über die halbe Länge des Adapters aus diesem gezogen werden kann und anschließend in dem Adapter verbleibt. Auch in dieser Position ist ein Lösen des medizinischen Instruments vom Adapter möglich. Sowohl das Adaptergehäuse 130 als auch die Kopplungselement 144, 146 und der nicht näher dargestellte Getriebestrang, welcher im Gehäuse 130 sitzt, können aus Plastik oder Metall gefertigt sein. Die Fixiervorrichtung 150 kann ebenfalls aus Kunststoff oder Metall gefertigt werden.

In der FIG. 4 ist die Steuer- und/oder Bedienvorrichtung 200 ohne eine obere Abdeckung und das berührungsempfindliche Display 210 dargestellt. Neben der Steuerplatine 230 ist eine Spülvorrichtung 240 erkennbar, welche Schläuche 242/244 für eine Saugspülung und/oder eine Druckspülung umfasst. Die Schläuche bzw. die darin sich befindende Spülflüssigkeit wird mittels einer Pumpe 246 angesogen bzw. gedrückt. Die Schläuche 242 bzw. 244 können beispielsweise mit dem in der FIG. 3a dargestellten Spüladapter 190 gekoppelt werden (der mit dem medizinischen Instrument 170 verbunden ist), welcher das Lumen des Schaftes 172 mit dem Schlauch verbindet. Anschließend kann über das berührungsempfindliche Display ein Programm initiiert werden, weiches eine Druck- bzw. Saugspülung des Lumen des Schaftes 172 ermöglicht.

Die Steuerung 230 ist derart programmiert, dass die einzige Pumpe 246 alle vier sich in der Adapterbank 120 befindlichen Instrumente mit einer Saug- bzw. Druckspülung beaufschlagen kann. Hierbei wird das Saugen bzw. Drücken kanalweise, das heißt ein Adapter nach dem anderen, durchgeführt, so dass zumindest für die Saugspülung festgestellt werden kann, ob das Lumen des Schaftes des medizinischen Instruments verstopft ist oder nicht. Die Umschaltung zwischen Saug- und Druckbetrieb wird durch einen Saug-/und Druckumschalter 248 durchgeführt. Die Kanalumschaltung, d.h. die kanalweise Beaufschlagung findet in einem nicht dargestellten Kanalumschalter statt. Die Verstopfung kann dabei über den durch die Pumpe tretenden Fluss bzw. den damit verbundenen Druck ermittelt werden. Diese Technik kommt teilweise auch in den Produkten Sonomic zum Einsatz.

Die Steuervorrichtung 230 umfasst, wie eingangs erwähnt, einen Prozessor und eine Speichereinheit. In der Speichereinheit sind verschiedene Programme zur Bewegung der Instrumente abgespeichert. So kann abhängig vom eingelegten Instrument ein Programm gewählt werden, welches den entsprechenden Winkelbereich der Drehung des Instruments und die anzusteuernden Kopplungselemente definiert. Diese Programme werden über den Prozessor initialisiert und der Prozessor übernimmt eine Steuerung des Antriebs, so dass dieser gemäß dem Programm die Bewegung der Kopplungselemente einleitet.

In der FIG. 4 ist ebenfalls ein Anschluss 250 erkennbar, über welchen eine Verbindung zwischen dem Antrieb 160 der Zusatzvorrichtung 100 mit der Bedien- und/oder Steuereinheit 200 stattfinden kann. Bei einer Verbindung kann es sich beispielsweise um eine Kupfer-Verbindung handeln.

## Patentansprüche

1. Zusatzvorrichtung (100) für eine Wanne (2) eines Ultraschallgeräts (1), welche ein Gestell (110) und mindestens einen Adapter (121 - 124) für ein medizinisches Instrument aufweist, wobei der Adapter ein Gehäuse (130) und mindestens ein mittels eines Antriebs (160) gegenüber dem Gehäuse bewegbares und mit dem medizinischen Instrument koppelbares Kopplungselement (140, 142; 144, 146) umfasst, so dass eine Bewegung des Kopplungselements eine aktive Bewegung zumindest eines Bereich des medizinischen Instruments bewirkt.

2. Zusatzvorrichtung nach Anspruch 1, wobei der Adapter eine bewegbare Fixierungsvorrichtung (150) zum Fixieren des medizinischen Instruments (170) umfasst.

3. Zusatzvorrichtung nach einem der vorhergehenden Ansprüche, wobei das Kopplungselement an einer dem medizinisches Instrument zugewandten Oberfläche eine Nut (148) zur Kopplung an das medizinische Instrument umfasst.

4. Zusatzvorrichtung nach einem der vorhergehenden Ansprüche, wobei das Kopplungselement federnd gelagert ist.

5. Zusatzvorrichtung nach einem der vorhergehenden Ansprüche, wobei das Kopplungselement rotierbar ist.

6. Zusatzvorrichtung nach einem der vorhergehenden Ansprüche, wobei das Kopplungselement mechanisch, hydraulisch oder pneumatisch mit dem Antrieb gekoppelt ist.

7. Zusatzvorrichtung nach einem der vorhergehenden Ansprüche, das Gestell derart ausgebildet ist, dass eine krafterzeugende Komponente des Antriebs außerhalb der Wanne und der mindestens eine Adapter innerhalb der Wanne angeordnet ist.

8. Zusatzvorrichtung nach einem der vorhergehenden Ansprüche, wobei das Gehäuse des Adapters gegenüber dem Gestell fixiert ist.

9. Zusatzvorrichtung nach einem der vorhergehenden Ansprüche, wobei das Gehäuse Öffnungen (125) aufweist und wasserdurchlässig ist.

10. Zusatzvorrichtung nach einem der vorhergehenden Ansprüche, wobei eine Saug- und/oder Druckspülungsvorrichtung zur Spülung des medizinischen Instruments vorhanden ist, wobei die Spülungsvorrichtung (240) einen Spüladapter (190) zur Kopplung an das medizinische Instrument und/oder einen Saug- und Druckutnschalter (248) umfasst.

11. Steuer- und/oder Bedienvorrichtung (200) für ein Ultraschallgerät (1) und zur Steuerung einer Zusatzvorrichtung (100) nach einem der vorhergehenden Ansprüche, wobei die Steuer- und/oder Bedienvorrichtung derart konfiguriert ist, dass eine vorzugsweise regelmäßige Bewegung des mindestens einen Kopplungselements durch den Antrieb bewirkt wird.

12. Steuer- und/oder Bedienvorrichtung nach Anspruch 11, wobei eine Spülvorrichtung (240) mit einem Saug- und Druckumschalter (248) vorhanden ist.

13. Verfahren zur aktiven Bewegung zumindest eines Bereich eines medizinischen Instruments in einer Wanne eines Ultraschallbads, wobei das medizinische Instrument in einen Adapter eingelegt wird und derart mit einem gegenüber einem Gehäuse beweglichen Kopplungselement des Adapters gekoppelt wird, dass eine Bewegung des Kopplungselements eine aktive Bewegung des Bereichs des medizinischen Instruments bewirkt.

14. Verfahren nach Anspruch 13, wobei das medizinische Instrument während der Beaufschlagung der Wanne mit Ultraschall bewegt wird.

15. Verfahren nach einem der Ansprüche 13 oder 14, wobei während der aktiven Bewegung des medizinischen Instruments eine Kavität des medizinischen Instruments eine Flüssigkeit des Ultraschallbads in die Kavität gesogen und/oder gedrückt wird.
